# EUROPEAN PATENT APPLICATION

(11) **EP 4 541 191 A1**
(43) Date of publication of application: **23.04.2025**
(21) Application number: 24188325.5
(22) Date of filing: 12.07.2024
(51) Int. Cl.: A01N 63/28, A01P 3/00, C12N 1/20

(54) **MARINE STREPTOMYCES AS NATURAL ANTIFUNGAL AGENTS TO COMBAT THE CHESTNUT BROWN ROT FUNGUS GNOMONIOPSIS SMITHOGILVYI**

(30) Priority: 13.07.2023 PT 2023118816
(71) Applicant: CIIMAR - Centro Interdisciplinar de Investigação Marinha e Ambiental, 4450-208 Matosinhos (PT); Universidade de Trás-os-Montes e Alto Douro, 5000-801 Vila Real (PT); Instituto Politécnico De Bragança, 5300-253 Bragança (PT); ARDITI - Agência Regional para o Desenvolvimento da Investigação, Tecnologia e Inovação - Associação / Mare, 9200-044 Funchal (PT)
(72) Inventor: MAGALHÃES CARVALHO, Maria De Fátima, Matosinhos (PT); RAMOS SAMPAIO, Ana Cristina, Vila Real (PT); GIRÃO SILVA MARTINS, Mariana, Matosinhos (PT); COELHO RIBEIRO, Inês Filipa, Matosinhos (PT); MAIOTO SANTOS, Rita, Penafiel (PT); SOUSA GOMES, Ana Filipa, Santa Marta de Penaguião (PT); DOS SANTOS ALFARELA, Cláudia, Vila Pouca de Aguiar (PT); AZEVEDO RODRIGUES, Paula Cristina, Bragança (PT); DE CAMPOS MUCHA, Ana Paula, Matosinhos (PT); DA SILVA COSTA, Maria Isabel, Matosinhos (PT); DOMINGUES BRAGA HENRIQUES, Andreia Filipa, Funchal (PT)
(74) Representative: Pereira da Cruz, Joao

(57) **Abstract**

This disclosure concerns *Streptomyces* spp. strains, isolated from different marine biotopes, able to inhibit the growth of *Gnomoniopsis smithogilvyi* (*syn. Gnomoniopsis castaneae*) (Gs). This fungus is the causal agent of chestnut brown rot responsible for damages in post-harvest fruits, and of wood cankers in the chestnut tree, and is responsible for massive losses in the chestnut sector. The actinobacterial extracts, as well as the corresponding strains, presented an excellent activity against Gs. These strains can be used in integrated and organic agriculture for biological control and integrated Gs management. This ability is of utmost importance in the context of the banning of several fungicidal substances.

## Description

### Field of the Invention

The present disclosure relates to fungicides and concerns marine *Streptomyces* spp. strains capable of inhibiting the growth of the fungus *Gnomoniopsis smithogilvyi* (syn. *Gnomoniopsis castaneae*) (Gs). This fungus is the causal agent of chestnut brown rot that damages the fruit after harvest, and of wood cankers in the chestnut tree.

### Background of the Invention

Fungicides used in agriculture account for about 16% of the global volume of pesticides and are very effective. Despite their effectiveness, they are highly toxic to aquatic life with permanent effects (Jørgensen and Heick, 2021). Thus, and especially in Europe, many azoles and phosphonates have been withdrawn or will soon be withdrawn, which could have a significant impact in the fight against fungal diseases, in the short and medium term. Although some antagonistic microorganisms such as bacteria or fungi have effectively reduced some diseases *in situ,* it is necessary to increase the diversity of these microorganisms, since their antagonistic action varies greatly with the antagonist strain and the target species. Companies producing agrochemicals will be the potential interested parties, which can amplify their biological fungicides offer to farmers.

Currently, a treatment against the fungus *Gnomoniopsis smithogilvyi* (syn. *Gnomoniopsis castaneae*) (Gs) that can be at the same time effective and environmentally friendly is not available. In Portugal, this fungus was recently characterized (Possamai et al., 2023). The limited options that are currently available to fight this fungus consist of synthetic fungicides that have a significant impact on the environment as well as in human and animal health, which, for this reason, will be prohibited.

The present invention provides a solution that uses marine actinobacterial strains affiliated with the genus *Streptomyces* as a natural and environmentally friendly solution to effectively prevent the development of Gs in chestnuts, avoiding the high economical losses resulting from this infection.

A product consisting of *Streptomyces* spp. strains or of its spores in chestnut production has several advantages when compared to current treatments. It replaces synthetic fungicides with natural biocontrol agents, which is a natural and environmentally friendly solution for solving a very significant agricultural problem: chestnut brown rot in the chestnut fruits. The use of microorganisms with a marine origin may limit their predominance in the native microbial communities.

Overall, this is an excellent solution to face the problem of the banning of fungicides currently in use to fight Gs. Additionally, these natural biocontrol agents are produced by an ecological process.

### General Description of the Invention

The present patent application relates to the use of *Streptomyces* spp. strains or of its spores as a fungicide and a process to obtain it.

In a preferred embodiment of the present disclosure an antimicrobial composition, preferably an antifungal composition, comprising the extracts/spores/cells of a *Streptomyces spp.* strain comprising one of the following genetic sequences: SEQ. ID No. 1 - SEQ. ID No. 13 or nucleic acid sequences with at least 95%, preferably 99% sequence homology thereto, wherein the concentration of said strain is between 1x105 - 5x107 CFU/mL, even more preferably 4x105 - 5x108 CFU/mL is disclosed.

In a further embodiment, it is disclosed an antimicrobial composition of the present disclosure comprising a suspension concentrate of said *Streptomyces* spp. strains.

In a further embodiment, it is disclosed a biocide comprising the composition of the present disclosure and a suitable carrier.

In a further embodiment, it is disclosed a fungicide comprising the composition of the present disclosure and a suitable carrier.

In a further embodiment the present disclosure encompasses a spray for treatment of plant crops, comprising the composition of the present disclosure.

In a further embodiment the present disclosure encompasses a process for obtaining the composition of the present disclosure comprising the steps of:
isolating a given *Streptomyces* spp. strain from a marine source, preferably macroalgae, deep-sea or coastal sediments;
cultivating said strains in a culture medium;
incubating said strains in the dark, preferably for at least 4 days;
adding a suitable resin to the culture medium;
incubating said culture medium comprising a resin and a biomass with said strains, preferably for at least 3 days;
separating the biomass and the resin of said culture medium, preferably by centrifugation;
washing and freeze-drying the obtained biomass and resin;
extracting and drying;
to obtain the composition of the present disclosure.

In a further embodiment, the present disclosure encompasses a process according to the present disclosure wherein step i) comprises the following steps:
supplementing said medium with cycloheximide, nystatin and nalidixic acid to obtain a culture;
cryopreserving said culture;
extracting DNA from said culture;
identifying *Streptomyces* spp. isolates by PCR and genetic sequencing, preferably by DNA sequencing.

In a further embodiment, the present disclosure encompasses a process according to the present disclosure, wherein the marine sediment is a coastal sediment or a deep-sea sediment.

In a further embodiment, the present disclosure encompasses a process, wherein the strain is isolated from the tissues of the macroalgae *Laminaria ochroleuca.*

In a further embodiment, the present disclosure encompasses a process, wherein the strain is isolated from the tissues of *Codium tomentosum.*

In a further embodiment, the present disclosure encompasses a process, wherein the strain is obtained from a deep-sea sediment and a deep-sea sponge, respectively.

In a further embodiment, the present disclosure encompasses the use of the composition of the present disclosure against a phytopathogenic fungi, particularly against the fungus *Gnomoniopsis smithogilvyi.*

In a further embodiment, the present disclosure encompasses a method of treating an affected area of a plant during the flowering of female flowers or in pre-harvest, preferably a chestnut tree, comprising the following steps:
obtaining the composition of the present disclosure;
applying the composition in a suitable form for covering the affected area, preferably by spraying.

In a further embodiment, the present disclosure encompasses a method, wherein the affected area is a chestnut fruit/chestnut brown rot.

None of the objects of the present disclosure are obtained exclusively by crossing and selection.

### Brief Description of Figures

**Figure 1****.** Inhibition zone diameters in mm (n=3) obtained for the tested extracts (15 µg) against Gs. Top: small dashed line - fluconazole (25 µg); middle dashed line - amphotericin B (10 µg). Bottom: Inhibition zone diameters for M4 (KENR13A), M5 (KENR18) and M6 (KENR25) extracts on Müeller-Hinton agar (MHA) front (left) and reverse (right) view.
**Figure 2****.** Confrontation assay of anti-Gs activity of the actinobacterial isolates 18A, MA3 2.14 and S_044 25, after 12 days of incubation at 25 °C, front (top) and reverse (bottom) Petri dish views.

### Detailed Description of the Invention

In the present disclosure, it is identified the *Streptomyces* spp. power to combat the fungus *Gnomoniopsis smithogilvyi,* which causes chestnut brown rot, that damages the fruit after harvest, and of wood cankers in the chestnut tree.

The aim of the present disclosure is to avoid the development of this fungus as it is highly detrimental for chestnut production, while also avoiding the massive waste of chestnuts infected with this fungus.

In an embodiment, the present disclosure encompasses a composition, such as a suspension concentrate (SC), comprising the spores or cells of *Streptomyces* spp. strains (18A, 18B, 8A1, 29, MA3 2.14, KenR13, KenR13A, KenR18, KenR25, CTR-58, CT-R49, S_0712.9 and S_044 25).

In a further embodiment, the present disclosure encompasses a spray comprising the composition of the previous paragraph to be applied on the crop, which has several advantages in chestnut production when compared to current chemical treatments. It replaces synthetic fungicides with natural biocontrol agents, an environmentally friendly solution for solving a very significant agricultural problem: chestnut brown rot in the chestnut fruits.

In a particular embodiment, which does not limit the scope of the present disclosure, the following *Streptomyces* strains are encompassed: [18A (accession number SEQ ID No. 1 (AN): MN134355 https://www.ncbi.nlm.nih.gov/nuccore/MN134355.1/), SEQ ID No. 2 18B (AN: MN134354 https://www.ncbi.nlm.nih.gov/nuccore/MN134354), SEQ ID No. 3 8A1 (AN: MN134348 https://www.ncbl.nlm.nih.gov/nuccore/MN134348), SEQ ID No. 4 29 (AN: MN134352 https://www.ncbi.nlm.nih.gov/nuccore/MN134352), SEQ ID No. 5 MA3 2.14 (AN: OQ363656 https://www.ncbi.nlm.nih.gov/nuccore/OQ363656), SEQ ID No. 6 S_0712.9 (AN: OR208592 https://www.ncbi.nlm.nih.gov/nuccore/OR208592), SEQ ID No. 7 S_044 25 (AN: OR211586 https://www.ncbi.nlm.nih.gov/nuccore/OR211586), SEQ ID No. 8 KenR13 (AN: MK254596 https://www.ncbi.nlm.nih.gov/nuccore/MK254596), SEQ ID No. 9 KenR13A (AN: MK254595 https://www.ncbi.nlm.nih.gov/nuccore/MK254595), SEQ ID No. 10 KenR18 (AN: MK254590 https://www.ncbi.nlm.nih.gov/nuccore/MK254590), SEQ ID No. 11 KenR25 (AN: MK254582 https://www.ncbi.nlm.nih.gov/nuccore/MK254582), SEQ ID No. 12 CTR-58 (AN: OR208768 - https://www.ncbi.nlm.nih.gov/nuccore/OR208768) and SEQ ID No. 13 CTR-49 (AN: OR208769 https://www.ncbi.nlm.nih.gov/nuccore/OR208769)], which were isolated from various marine sources (macroalgae, deep-sea and coastal sediments), and identified as *Streptomyces* spp.

The present disclosure has surprisingly identified that the strains have excellent antifungal properties against several Gs isolates.

### Example 1

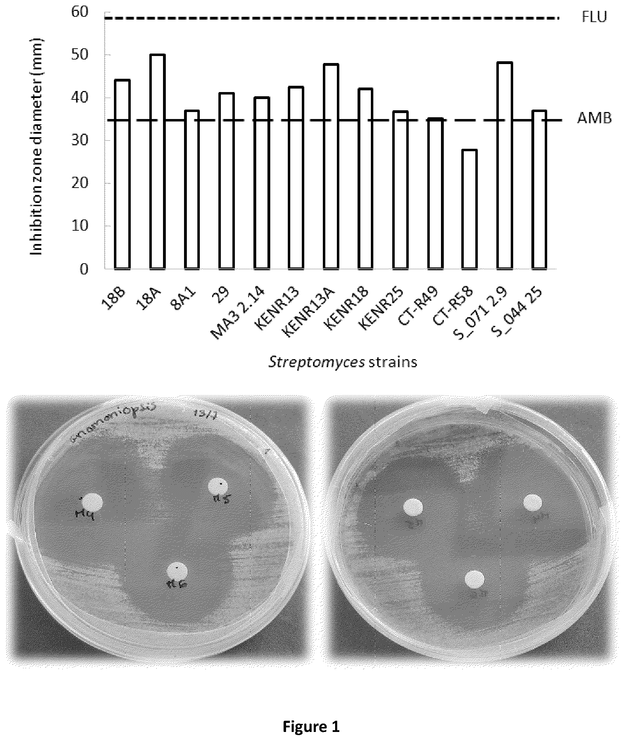

The extracts of these isolates presented a significant anti-Gs activity in the diffusion plate assay, comparable or higher than the inhibition caused by Fluconazole (FLU) and Amphotericin B (Figure 1, Table 1), two common antifungal agents against clinical isolates with distinct mechanisms of action.

**Table 1 - Global values of the inhibition zones (IZ) diameters (mm) for the 12 extracts tested against three strains of Gs, and IZ values for fluconazole (FLU, 25 µg) and Amphotericin B (AMB, 10 µg).**

| | |
|---|---|
| Minimum | 22.0 |
| Maximum | 60.0 |
| Average | 41.5 |
| Median | 42.2 |
| FLU | 59.0 |
| AMB | 35.0 |

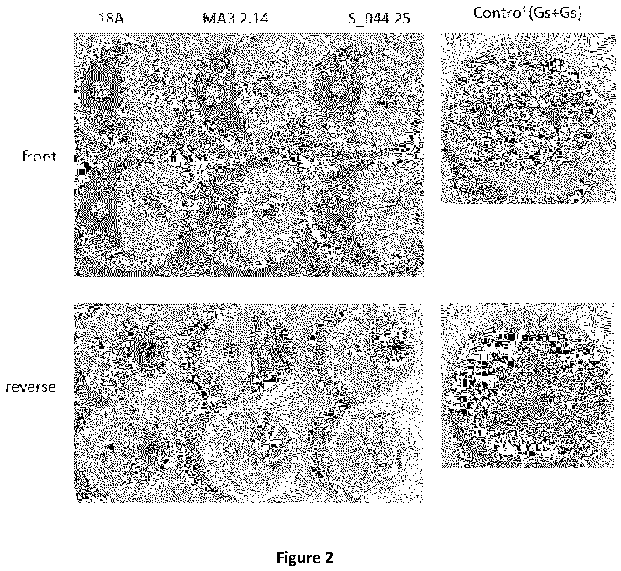

The confrontation tests between each actinobacterial isolate (n=5) and Gs strains (n=3) confirmed the capacity of directly inhibiting fungal growth. The lowest inhibition percentage achieved was 33.2% and the highest 73.4% for the tested bacterial suspensions. Figure 2 shows some results of the dual confrontation test between Gs and three *Streptomyces* isolates (18A, MA3 2.14 and S_044 25), where it is evident the antagonism of the bacteria after 12 days of incubation. Although some fungicides are known for controlling Gs, such as azoles (e.g., tebuconazole, a triazole, used against phytopathogenic fungi) and phosphonates, the prohibition of these substances, due to environmental safety and human and animal health, calls for better alternatives, namely natural compositions with improved effect against Gs.

### Example 2

Thirteen *Streptomyces* strains isolation: Strains 18A, 18B, 8A1 and 29 were isolated from a coastal sediment collected at a depth of 0.15 m in the intertidal zone of a sandy beach located in Parque Natural do Litoral Norte (Cepães beach), in northern Portugal (Ribeiro et al., 2020). Strains KENR13, KENR13A, KENR18 and KENR25 were isolated from the tissues of the macroalgae *Laminaria ochroleuca* and strains CT-R58 and CT-R49 from the tissues of *Codium tomentosum,* both specimens collected in Mindelo Beach, in the northern Portuguese coast (Girão et al., 2019). Strain MA3 2.14 was obtained from a deep-sea sediment, and strains S_071 2.9 and S_044 25 were isolated from two deep-sea sponges, all collected in Madeira Archipelago, between 650 - 2300 m depth (Ribeiro et al., 2023). Different pre-treatments and culture media were used for the isolation of these strains (Table 2). Strains KENR13, KENR13A, KENR18, KENR25 and CT-R58 were isolated and purified using SCN medium (Starch-Casein-Nitrate agar; per liter of deionized water: 10 g of soluble starch, 0.3 g of casein, 2 g of K₂HPO₄, 2 g of KNO₃, 2 g of NaCl, 0.05 g of MgSO₄.7H₂O, 0.02 g of CaCO₃, 0.01 g of FeSO₄.7H₂0 and 17 g of agar). Strains CT-R49, 18A, 18B, 8A1 and 29 were retrieved in NPS medium (Nutrient-Poor-Sediment agar; per liter of seawater: 100 mL of marine sediment extract obtained by washing 900 mL of sediments with 500 mL of seawater and 17 g of agar), strains MA3 2.14 and S_044 25 were obtained using M1 medium (per liter of seawater: 10 g of soluble starch, 4 g of yeast extract, 2 g of peptone and 17 g of agar) and strain S_071 2.9 was isolated from the RH medium (per liter of seawater: 4 g of chitin and 17 g of agar).

**Table 2 - Pre-treatments and culture media used for the isolation of Actinobacteria.**

| **STRAINS** | **SOURCE** | **PRE-TREATMENT** | **CULTURE MEDIA** |
|---|---|---|---|
| 18A | Coastal sediment | Water bath (60 °C for 5 min) | NPS |
| 18B | | | |
| 29 | | | |
| 8A1 | Coastal sediment | Antibiotics (20 ppm) + Water bath (28 °C for 30 min) | NPS |
| MA3 2.14 | Deep-sea sediment | Water bath (60 °C for 30 min) | M1 |
| S_071 2.9 | Deep-sea sponge | Water bath (57 °C for 15 min) | RH |
| S_044 25 | Deep-sea sponge | Water bath (57 °C for 15 min) | M1 |
| KENR13 | Macroalgae *Laminaria ochroleuca* | Tissue surface disinfection with CTAB buffer and proteinase K Water bath (60 °C for 30 min) | SCN |
| KENR13A | | | |
| KENR18 | | | |
| KENR25 | | | |
| CT-R58 | Macroalgae *Codium tomentosum* | Water bath (60 °C for 30 min) | SCN |
| CT-R49 | Macroalgae *Codium tomentosum* | Water bath (60 °C for 30 min) | NPS |

All media were supplemented with cycloheximide (50 mg/L), nystatin (50 mg/L) and nalidixic acid (50 mg/L).

Pure cultures were cryopreserved at -80 °C in 30% (v/v) of glycerol.

As to what regards the identification as *Streptomyces* spp.: all isolates were taxonomically identified through 16S rRNA gene sequencing. Biomass for DNA extraction was obtained by growing each isolate at 28 °C, for one week, in the corresponding isolation agar media, with the exception of those obtained from the coastal sediment that were grown on ISP2 culture medium (per liter of seawater: 4 g of yeast extract, 10 g of malt extract, 4 g of glucose and 17 g of agar). Genomic DNA was extracted using the E.Z.N.A. Bacterial DNA Kit (Omega Bio-Tek, GA, United States) according to the manufacturer's instructions. The 16S rRNA gene was amplified by PCR using the universal primers 1492R (5'-GGTTACCTTGTTACGACTT-3') and 27F (5'-GAGTTTGATCCTGGCTCAG-3') and the sequence compared to EzBiocloud and NCBI BLAST databases to establish the final identification. The PCR mixture (total volume of 10 µL) contained 5 µL of Taq PCR Master Mix (Qiagen, Valencia, CA, USA), 1 µL of primer 1492R (2 µM), 1 µL of primer 27F (2 µM) and 3 µL of DNA template. PCR conditions were as follows: initial denaturation at 95 °C for 15 min, followed by 30 cycles at 94 °C for 30 s, 48 °C for 90 s, 72 °C for 90 s and a final extension at 72 °C for 10 min.

In an embodiment, the extracts of these isolates were obtained in the following manner: In order to obtain the crude extracts, each strain was grown in a 100 mL Erlenmeyer flask containing 30 mL of liquid culture medium (with the same composition of the isolation medium, but without agar). The flasks were incubated at 28 °C, 100 rpm, in the dark. After 4 days of incubation, 0.5 g of Amberlite XAD16N resin (Sigma-Aldrich, MO, United States) were added to the culture and incubation continued for three additional days. The biomass and resin were centrifuged at 3,600 g for 10 min, washed twice with dH₂O and freeze-dried. The freeze-dried biomass and resin were then extracted with 30 mL of a solution of acetone/methanol (1:1 (v/v)). The organic layer was recovered and dried in a rotary evaporator.

The resulting extract was used in the bioassays, namely:
- Tests that prove significant anti-Gs activity (Figure 1): Plate diffusion method: Petri dishes (90 mm diameter) with 4.0 mm depth of Mueller-Hinton Agar (MHA) medium (Matuschek et al., 2014), were inoculated with a standardized spore suspension (4x10⁵ - 5x10⁶ CFU/mL), obtained according to (Kulkarni et al., 2018). Then, filter paper discs were placed on the agar surface and saturated with 15 µL of each extract (3 replicates per extract). After incubation at 28 °C for 24 h, the diameter (in mm) of the growth inhibition zone was read.
- The confrontation tests between each actinobacterial isolate (n=5) and Gs (Figure 2) which confirmed the capacity of inhibiting fungal growth: *In vitro* confrontation tests were performed on 4 native isolates of Gs and actinobacteria, using a modified version of the method of Larralde et al., (2008). On Petri dishes containing potato dextrose agar (PDA), bacteria and Gs were confronted face to face and separated by 5 cm: Gs by placing a 6 mm diameter circle of active mycelium and a bacterial suspension (10 µL of 1.0 McFarland scale); they were incubated at 25 + 2 °C (in triplicate, for each Gs strain and antagonist) and the growth measured until day 6. The inhibition percentage (IP) of growth was monitored every 48 hours using the following formula: IP =[(R1-R2)/R1] x100, where R1 is the radial growth of the non-confronted strain of Gs (control), and R2 is the radial growth of Gs confronted against bacteria.

Overall, this is an excellent solution to face the problem of the banning of fungicides currently in use to fight Gs. Additionally, these natural biocontrol agents are produced by an ecological process.

These strains, and the substances they produce, sustainably control Gs, and thus can be used in integrated and organic agriculture for biological control and in integrated Gs management.

### REFERENCES

Girão, M., Ribeiro, I., Ribeiro, T., Azevedo, I. C., Pereira, F., Urbatzka, R., Leão, P. N., & Carvalho, M. F. (2019). Actinobacteria isolated from Laminaria ochroleuca: A source of new bioactive compounds. Fronteirs in Microbiolology, 10, 683.
Jørgensen, L. N., Heick, T. M. (2021). Azole use in agriculture, horticulture, and wood preservation - is it indispensable? Frontiers in Cellular and Infection Microbiology, 11: 730297
Kulkarni, S.S., Bhakre, J. B., Damle, A.S. (2018). In vitro susceptibility testing of four antifungal drugs against fungal isolates in onychomycosis. International Journal of Research in Medical Sciences, 6(8), 2774. doi: 10.18203/2320-6012.ijrms20183268
Larralde-Corona, C.P., Santiago-Mena, M.R., Sifuentes-Rincón, A.M., Rodriguez-Lina, I.C., Rodriguez-Pérez, M.A., Shirai, K., Narváez-Zapata, J.A. (2008). Biocontrol potential and polyphasic characterization of novel native Trichoderma strains against Macrophomina phaseolina isolated from sorghum and common bean. Applied Microbiology and Biotechnology, 80: 167-177. doi:10.1007/s00253-008-1532-0
Matuschek, E., Brown, D.F.J., Kahlmeter, G. (2014). Development of the EUCAST disk diffusion antimicrobial susceptibility testing method and its implementation in routine microbiology laboratories. Clinical Microbiology and Infection, 20(4), O255-O266. doi: 10.1111/1469-0691.12373
Possamai, G., Dallemole-Giaretta, R., Gomes-Laranjo, J., Sampaio, A., Rodrigues, P. (2023). Chestnut brown rot and Gnomoniopsis smithogilvyi: characterization of the causal agent in Portugal. Journal of Fungi. 9(4):401. doi: 10.3390/jof9040401.
Ribeiro, I., Antunes, J., Alexandrino, D., Tomasino, M., Almeida, E., Hilário, A., Urbatzka, R., Leão, P., Mucha, A., & Carvalho, M. (2023). Actinobacteria from Arctic and Atlantic deep-sea sediments-Biodiversity and bioactive potential. Fronteirs in Microbiology, 14, 1158441.
Ribeiro, I., Girão, M., Alexandrino, D. A. M., Ribeiro, T., Santos, C., Pereira, F., Mucha, A. P., Urbatzka, R., Leão, P. N., & Carvalho, M. F. (2020). Diversity and Bioactive Potential of Actinobacteria Isolated from a Coastal Marine Sediment in Northern Portugal. Microorganisms, 8(11). doi:10.3390/microorganisms8111691

## Claims

1. Antimicrobial composition, preferably an antifungal composition, comprising the extracts/spores/cells of a *Streptomyces* spp. strain comprising one of the following genetic sequences: SEQ. ID No. 1 - SEQ. ID No. 13 or nucleic acid sequences with at least 95%, preferably 99% sequence homology thereto, wherein the concentration of said strain is between 1x10⁵ - 5x10⁷ CFU/mL, even more preferably 4x10⁵ - 5x10⁸ CFU/mL.

2. Antimicrobial composition according to the previous claim comprising a suspension concentrate of said *Streptomyces* spp. strains.

3. Biocide comprising the composition of claims 1-2 and a suitable carrier.

4. Fungicide comprising the composition of claims 1-2 and a suitable carrier.

5. Process for obtaining the composition of claims 1-2 comprising the steps of:
i) isolating a given *Streptomyces* spp. strain from a marine source, preferably macroalgae, deep-sea or coastal sediments;
ii) cultivating said strains on a culture medium;
iii) incubating said strains in the dark, preferably for at least 4 days;
iv) adding a suitable resin to the culture medium;
v) incubating said culture medium comprising a resin with said strains, preferably for at least 3 days;
vi) Separating the biomass and the resin from the said culture medium, preferably by centrifugation;
vii) washing and freeze-drying the obtained biomass and resin;
viii) extracting a dried biomass and resin;
to obtain the composition of claims 1- 2.

6. Process according to the previous claim wherein step i) comprises the following steps:
pre-treating with selective culture media supplemented with cycloheximide,
nystatin and nalidixic acid to obtain pure cultures;
cryopreserving said cultures;
extracting DNA from said cultures;
identifying the cultures as *Streptomyces spp.* by PCR and genetic sequencing,
preferably DNA sequencing.

7. Process according to any one of claims 5-6, wherein the marine sediment is a coastal sediment or a deep-see sediment.

8. Process according to any one of claims 5-7, wherein the strain is isolated from the tissues of macroalgae *Laminaria ochroleuca.*

9. Process according to any one of claims 5-7, wherein the strain is isolated from the tissues of *Codium tomentosum.*

10. Process according to any one of claims 5-7, wherein the strain is obtained from a deep-sea sediment or deep-sea sponges.

11. Use of the composition of any one of claims 1-2 for treatment of a phytopathogenic fungi, particularly against *Gnomoniopsis smithogilvyi.*

12. Method of treating an affected area of a plant, preferably a chestnut, during the flowering of the female flowers or in pre-harvest, comprising the following steps:
i) obtaining the composition of claim 1;
ii) applying the composition in a suitable form for covering the affected area, preferably by spraying.

13. Method according to the previous claim, wherein the affected area is preferably a chestnut fruit /chestnut brown rot.
